# EUROPEAN PATENT SPECIFICATION

(11) **EP 3 344 175 B1**
(45) Date of publication and mention of the grant of the patent: **24.07.2019**
(21) Application number: 16770447.7
(22) Date of filing: 31.08.2016
(51) Int. Cl.: A61B 17/88, A61B 17/17, A61B 17/70, A61B 34/30, B21D 7/00, A61B 46/27, A61B 46/10, A61B 34/20, A61B 90/57, A61B 34/10, A61B 90/00, A61B 90/50

(54) **ROBOTIC SURGICAL SYSTEMS FOR SPINAL ROD BENDING**
ROBOTISCHE CHIRURGIESYSTEM ZUM KRÜMMEN SPINALER STANGEN
SYSTÈMES CHIRURGICAUX ROBOTIQUES POUR CINTRER LA TIGE SPINALE

(30) Priority: 31.08.2015 US 201562212550 P
(43) Date of publication of application: 11.07.2018
(73) Proprietor: KB Medical SA, 1024 Ecublens (CH)
(72) Inventor: KOSTRZEWSKI, Szymon, 1003 Lausanne (CH); CHAPPUIS, Oliver, Le Chatelard 1095 Lutry (CH)
(74) Representative: Morabito, Sara
(86) International application number: PCT/EP2016/070515
(87) International publication number: WO 2017/037113

(56) References cited:
- WO-A1-2014/088801
- WO-A1-2015/054543
- US-A1- 2012 247 173
- US-A1- 2015 100 066
- US-B1- 8 601 923
- US-B2- 7 957 831

## Description

### BACKGROUND OF THE INVENTION

Robotic-assisted surgical systems have been developed to improve surgical precision and enable the implementation of new surgical procedures. For example, robotic systems have been developed to sense a surgeon's hand movements and translate them to scaled- down micro-movements and filter out unintentional tremors for precise microsurgical techniques in organ transplants, reconstructions, and minimally invasive surgeries. Other robotic systems are directed to telemanipulation of surgical tools such that the surgeon does not have to be present in the operating room, thereby facilitating remote surgery. Feedback- controlled robotic systems have also been developed to provide smoother manipulation of a surgical tool during a procedure than could be achieved by an unaided surgeon.

However, widespread acceptance of robotic systems by surgeons and hospitals is limited for a variety of reasons. Current systems are expensive to own and maintain. They often require extensive preoperative surgical planning prior to use, and they extend the required preparation time in the operating room. They are physically intrusive, possibly obscuring portions of a surgeons field of view and blocking certain areas around the operating table, such that a surgeon and/or surgical assistants are relegated to one side of the operating table. Current systems may also be non-intuitive or otherwise cumbersome to use, particularly for surgeons who have developed a special skill or "feel" for performing certain maneuvers during surgery and who find that such skill cannot be implemented using the robotic system. Finally, robotic surgical systems may be vulnerable to malfunction or operator error, despite safety interlocks and power backups.

One of the most popular methods of bone stabilization involves placement of screws in the bone and joining heads of the screws with rods. Some surgical techniques involve usage of different tools, such as a pedicle finder or K-wires. Such procedures rely strongly on the expertise of the surgeon, and there is significant variation in success rate among different surgeons. Screw misplacement is a common problem in such surgical procedures.

As shown in the example depicted in FIG. 2, rods can be bent to allow correction of the spine and adapt to positions of the screws head in the vertebrae. Rods are made of resistant materials (e.g. Titanium alloys, Chrome-Cobalt alloys) and require significant force to bend manually. Additionally, it is hard to manually recreate the desired two-dimensional and three-dimensional pattern, such as the bends shown FIG. 3. Further, as shown in FIG. 4, manual bending can lead to undesirable features that decrease the integrity and mechanical strength of the rods. Manual bending involves tools which may create curbs on the rods which decreases their mechanical strength. Multiple bending corrections can further weaken material. Prior art WO2015/054543 A1, US8601923 B1 and US7957831 B2 disclose a method for surgical spinal correction and a rod cutter.

Thus, it is desirable to have a way to bend rods in the operating room to greatly simplify the surgery and improve mechanical properties (e.g., strength and/or curvature) of the rods and thus outcomes for the patients.

### SUMMARY OF THE INVENTION

The disclosed technology relates to a rod bending module for use with a robotic surgical system in an operating room. The system which is capable to bend rods for surgeries directly in the operating room. The rigidity of the rods is such that the robotic arm alone would have to be large and unwieldy in an operating room to provide sufficient forces and torques. This invention introduces a bending module integrated with a robotic system which allows free bending of rods within limits required for surgeries. Additionally other technologies, such as navigation and automatic diagnosis algorithms defining ideal curves (e.g., spinal or other bone curves), can be integrated with the robotic surgical system.

The bending module, rod fixation and robotic system can be put together in different architectures. For example, the bending module may be mounted for example, on the mobile cart. It can be entirely external (e.g., including the actuator) to the cart (e.g., and the robotic surgical system) and sterilizable and/or autoclavable. The rod fixation device can be attached to the end effector of the robot such that the robot can position and rod in the bending module. This allows the robotic arm to move the rod in the bending module so that the rod can be bent at the appropriate locations. The bending module can be partially located inside the robotic surgical system. For example, the actuator for the bending module can be located in the mobile cart such that the motor does not need to be sterile. A sterile component would extend from the cart (or be extendable from the cart when activated) with an appropriate seal to maintain the sterile zone. The bending module can attach to the outside of the mobile cart and the bending die can be activated by the sterile component of the actuator that extends from (or can extend from) the mobile cart. In other embodiments, the bending module is affixed to the end effector and the rod fixation apparatus is attached to, for example, the mobile cart.

According to the invention, there is provided an apparatus according to the appended independent claim. Preferred embodiments are given in the dependent claims. In one aspect, the disclosed technology includes a machine for intraoperative bending of rods, the machine including: a bending apparatus for bending a rod, the bending apparatus comprising: a force die; a bend die; and a force transfer device that transfers energy from an actuator to the force die thereby causing a rod positioned between the force die and the bend die to bend around the bend die; and a fixation apparatus for releasably securing the bending apparatus to a robotic surgical system.

In certain embodiments, the fixation apparatus is magnetic, electro-magnetic, and/or mechanical (e.g. using lever device).

In certain embodiments, the fixation apparatus comprises at least one of push clips, push in rivets, screw rivets, clips, and tabs configured to attach to an interface on the robot.

In certain embodiments, the fixation apparatus comprises one or more holes sized for one or more bolts to pass through to secure the bending apparatus to the robotic surgical system via the one or more bolts.

In certain embodiments, the bending device is a ram bending device, three-roll bending device, compression bending device, or rotary draw bending device.

In certain embodiments, the bending device is a ram bending device, the force die comprises a first counter die and a second counter die, and the bend die is a radius block.

In certain embodiments, the bending device is a three-roll bending device, the force die comprises a first counter roller and a second counter roller, and the bend die is a bend roller.

In certain embodiments, the bending device is a compression bending device, the force die comprises a stationary bend die, the bending device comprises a clamp, and the bend die is a compression die.

In certain embodiments, the bending device is a rotary draw bending device, the force die comprises a stationary pressure die, the bending device comprises a clamp, a follower slide, and a wiper die, and the bend die is a rotatable bend die.

In certain embodiments, the bending module is a passive bending module.

In certain embodiments, the bending device comprises an actuator for applying a force to the force die thereby causing a rod positioned between the force die and the bend die to bend around the bend die.

In certain embodiments, the force transfer device is arranged to transfer a force from an actuator to the force die.

In certain embodiments, the bending module is an active bending module.

In certain embodiments, the bending module comprises a cutter for cutting rods.

In certain embodiments, the bending module is at least in part a fixed module.

In certain embodiments, the bending module is arranged to be releasably connected to a mobile cart.

In certain embodiments, the bending module is a floating module.

In certain embodiments, the bending module is arranged to be releasably connected to a robotic arm.

In certain embodiments, the actuator is separate from the bending apparatus. In certain embodiments, the actuator is internal to a mobile cart.

In certain embodiments, the bending apparatus is autoclavable. In certain embodiments, the bending apparatus is sterilizable.

In certain embodiments, the bending apparatus has at least one of a length, width, and height from 5.08 cm to 10.16 cm, 10.16 cm to 15.24 cm, 15.24 cm to 20.32 cm, 20.32 cm to 30.48 cm, and 30.48 cm to 60.96 cm.

In certain embodiments, the actuator is external to the bending module. In certain embodiments, the fixation apparatus comprises a lever device.

In another aspect, the disclosed technology includes a robotic surgical system for use in a surgical procedure, the system including: a robotic arm comprising an end-effector; an actuator for controlled movement of the robotic arm and positioning of the end effector; and a processor and a non-transitory computer readable medium storing instructions thereon wherein the instructions, when executed, cause the processor to: receive (e.g., determine) a desired curvature of a skeletal structure (e.g., spine, hip, leg, femur, tibia, fibia, hip, knee, or ankle) of a patient; determine a position of each the two or more screws in the patient during the surgical procedure (e.g., using a pointing device and a navigation system; e.g., wherein the position of each of the two or more screws is different than an ideal position of each screw); and intraoperatively determine the desired curvature of an implantable rod (e.g., for use in joining the heads of two or more screws) based at least in part on the desired curvature of the skeletal structure and the position of the two or more screws placed in the patient during the surgical procedure.

In certain embodiments, the instructions, when executed by the processor, cause the processor to: position the end effector thereby positioning the rod relative to a bending apparatus; and send signals to the bending apparatus that cause the bending apparatus to bend the rod.

In certain embodiments, the instructions, when executed by the processor, cause the processor to: position the end effector and send signals to a bending apparatus thereby causing the bending apparatus to bend the rod, thereby creating a shaped rod.

In certain embodiments, the instructions, when executed by the processor, cause the processor to: receive (e.g., determine) a desired curvature of a skeletal structure of a patient; determine a position of each the two or more screws in the patient during the surgical procedure (e.g., using a pointing device and a navigation system; e.g., wherein the position of each of the two or more screws is different than an ideal position of each screw); and intraoperatively determine the desired curvature of an implantable rod (e.g., for use in joining the heads of two or more screws) based at least in part on the desired curvature of the skeletal structure and the 20 position of the two or more screws placed in the patient during the surgical procedure.

In certain embodiments, the desired curvature of the skeletal structure is determined preoperatively.

In certain embodiments, the position of each of the two or more screws in the patient is determined intra-operatively.

In certain embodiments, the position of each of the two or more screws in the patient is determined using a navigation system (e.g., separate from or integrated into the robotic surgical system)
In certain embodiments, the position of each of the two or more screws in the patient is determined using a point device with the navigation system to identify the locations of the screws during the surgical procedure.

In certain embodiments, the instructions, when executed by the processor, cause the processor to send one or more signals to the bending apparatus to cause the bending apparatus to bend the rod to produce the shaped rod.

In certain embodiments, the shaped rod is shaped to connect the two or more screws to each other.

In certain embodiments, the system includes a rod fixation apparatus for grasping a rod (e.g., wherein the rod can be placed into the rod fixation "touch-free" - e.g., with a surgeon physically grasping the rod with his/her hand).

In certain embodiments, the rod fixation apparatus is arranged to be held by the end effector.

In certain embodiments, the end effector is a force and/or torque control end-effector.

In certain embodiments, the end effector is configured to hold a first surgical tool.

In certain embodiments, the end-effector comprises a tool holder attached to the robotic arm via a force sensor, wherein the tool holder is sized and shaped to hold a first surgical tool.

In certain embodiments, the system includes a manipulator configured to allow robotically-assisted or unassisted positioning and/or movement of the end-effector by a user with at least four degrees of freedom.

In certain embodiments, the system includes a handle extending from the end effector that may be grasp by a hand of a user to move and/or position the end effector.

In certain embodiments, the system includes a force sensor located between the robotic arm and the tool holder for measuring forces and/or torques applied by a user to the first surgical tool held by the tool holder.

In certain embodiments, the system includes a sensor that detects the presence of the hand of the user on the handle.

In certain embodiments, the robotic surgical system is configured to permit a surgeon to manually move the end-effector to a position for an operation.

In certain embodiments, the system includes: a bending apparatus for bending a rod, the bending apparatus comprising: a force die; a bend die; and a force transfer device that transfers energy from an actuator (e.g., external to the bending module) to the force die
thereby causing a rod positioned between the force die and the bend die to bend around the bend die; and a fixation apparatus for releasably securing the bending apparatus to a robotic surgical system.

In certain embodiments, the fixation apparatus is magnetic, electro-magnetic, and/or mechanical (e.g. using lever device).

In certain embodiments, the fixation apparatus comprises at least one of push clips, push in rivets, screw rivets, clips, and tabs configured to attach to an interface on the robot.

In certain embodiments, the fixation apparatus comprises one or more holes sized for one or more bolts to pass through to secure the bending apparatus to the robotic surgical system via the one or more bolts.

In certain embodiments, the bending device is a ram bending device, three-roll bending device, compression bending device, or rotary draw bending device.

In certain embodiments, the bending device is a ram bending device, the force die comprises a first counter die and a second counter die, and the bend die is a radius block.

In certain embodiments, the bending device is a three-roll bending device, the force die comprises a first counter roller and a second counter roller, and the bend die is a bend roller In certain embodiments, the bending device is a compression bending device, the force die comprises a stationary bend die, the bending device comprises a clamp, and the bend die is a compression die.

In certain embodiments, the bending device is a rotary draw bending device, the force die comprises a stationary pressure die, the bending device comprises a clamp, a follower slide, and a wiper die, and the bend die is a rotatable bend die.

In certain embodiments, the bending module is a passive bending module.

In certain embodiments, the bending device comprises an actuator for applying a force to the force die thereby causing a rod positioned between the force die and the bend die to bend around the bend die

In certain embodiments, the force transfer device is arranged to transfer a force from an actuator to the force die.

In certain embodiments, the bending module is an active bending module.

In certain embodiments, the bending module comprises a cutter for cutting rods. In certain embodiments, the bending module is at least in part a fixed module.

In certain embodiments, the bending module is arranged to be releasably connected to a mobile cart.

In certain embodiments, the bending module is a floating module.

In certain embodiments, the bending module is arranged to be releasably connected to a robotic arm.

In certain embodiments, the actuator is separate from the bending apparatus.

In certain embodiments, the actuator is internal to a mobile cart.

In certain embodiments, the bending apparatus is autoclavable. In certain embodiments, the bending apparatus is sterilizable.

In certain embodiments, the bending apparatus has at least one of a length, width, and height from 5.08 cm to 10.16 cm, 10.16 cm to 15.24 cm, 15.24 cm to 20.32 cm, 20.32 cm to 30.48 cm, and 30.48 cm to 60.96 cm.

In certain embodiments, the surgery is orthopedic surgery or spinal surgery.

In certain embodiments, the end-effector is configured to releasably hold the first surgical tool, allowing the first surgical tool to be removed and replaced with a second surgical tool. In certain embodiments, the manipulator is configured to allow robotically assisted of unassisted positioning and/or movement of the end-effector by a user with at least six degrees of freedom, wherein the six degrees of freedom are three degrees of translations and three degrees of rotations.

In certain embodiments, the patient position is a position of one or more markers placed in spatial relation to one or more vertebrae.

In certain embodiments, controlling the actuator to move the end-effector comprises controlling the actuator to move the end-effector in a direction corresponding to a direction of application of the force and/or torque.

In certain embodiments, the end-effector is configured to move at a predetermined measured pace upon application and detection of user force and/or torque applied to the end-effector in excess of the predetermined minimum force and/or torque and the predetermined measured pace is a steady, slow velocity.

In another aspect, the disclosed technology includes a robotic surgical system for performing surgery, the system including: a robotic arm comprising an end-effector; an actuator for controlled movement of the robotic arm and positioning of the end effector; and a processor and a non-transitory computer readable medium storing instructions thereon wherein the instructions, when executed, cause the processor to: intraoperatively coordinate the bending of an implantable rod to produce a shaped rod based at least in part on a desired curvature of a skeletal structure and the position of each of two or more screws in the patient during the surgical procedure.

In certain embodiments, the instructions, when executed by the processor, cause the processor to: position the end effector thereby positioning the rod relative to a bending apparatus; and send signals to the bending apparatus that cause the bending apparatus to bend the rod.

In certain embodiments, the instructions, when executed by the processor, cause the processor to: position the end effector and send signals to a bending apparatus thereby causing the bending apparatus to bend the rod, thereby creating a shaped rod.

In certain embodiments, the instructions, when executed by the processor, cause the processor to: receive (e.g., determine) a desired curvature of a skeletal structure of a patient; determine a position of each the two or more screws in the patient during the surgical procedure (e.g., using a pointing device and a navigation system; e.g., wherein the position of each of the two or more screws is different than an ideal position of each screw); and intraoperatively determine the desired curvature of an implantable rod (e.g., for use in joining the heads of two or more screws) based at least in part on the desired curvature of the skeletal structure and the position of the two or more screws placed in the patient during the surgical procedure.

In certain embodiments, the desired curvature of the skeletal structure is determined preoperatively.

In certain embodiments, the position of each of the two or more screws in the patient is determined intra-operatively.

In certain embodiments, the position of each of the two or more screws in the patient is determined using a navigation system (e.g., separate from or integrated into the robotic surgical system).

In certain embodiments, the position of each of the two or more screws in the patient is determined using a point device with the navigation system to identify the locations of the screws during the surgical procedure.

In certain embodiments, the instructions, when executed by the processor, cause the processor to send one or more signals to the bending apparatus to cause the bending apparatus to bend the rod to produce the shaped rod.

In certain embodiments, the shaped rod is shaped to connect the two or more screws to each other.

In certain embodiments, the system includes a rod fixation apparatus for grasping a rod (e.g., wherein the rod can be placed into the rod fixation "touch-free" - e.g., with a surgeon physically grasping the rod with his/her hand).

In certain embodiments, the rod fixation apparatus is arranged to be held by the end effector. In certain embodiments, the end effector is a force and/or torque control end-effector.

In certain embodiments, the end effector is configured to hold a first surgical tool.

In certain embodiments, the end-effector comprises a tool holder attached to the robotic arm via a force sensor, wherein the tool holder is sized and shaped to hold a first surgical tool.

In certain embodiments, the system includes a manipulator configured to allow robotically-assisted or unassisted positioning and/or movement of the end-effector by a user with at least four degrees of freedom.

In certain embodiments, the system includes a handle extending from the end effector that may be grasp by a hand of a user to move and/or position the end effector.

In certain embodiments, the system includes a force sensor located between the robotic arm and the tool holder for measuring forces and/or torques applied by a user to the first surgical tool held by the tool holder.

In certain embodiments, the system includes a sensor that detects the presence of the hand of the user on the handle.

In certain embodiments, the robotic surgical system is configured to permit a surgeon to manually move the end-effector to a position for an operation.

In certain embodiments, the system includes: a bending apparatus for bending a rod, the bending apparatus comprising: a force die; a bend die; and a force transfer device that transfers energy from an actuator (e.g., external to the bending module) to the force die thereby causing a rod positioned between the force die and the bend die to bend around the bend die; and a fixation apparatus for releasably securing the bending apparatus to a robotic surgical system.

In certain embodiments, the fixation apparatus is magnetic, electro-magnetic, and/or mechanical (e.g. using lever device).

In certain embodiments, the fixation apparatus comprises at least one of push clips, push in rivets, screw rivets, clips, and tabs configured to attach to an interface on the robot.

In certain embodiments, the fixation apparatus comprises one or more holes sized for one or more bolts to pass through to secure the bending apparatus to the robotic surgical system via the one or more bolts.

In certain embodiments, the bending device is a ram bending device, three-roll bending device, compression bending device, or rotary draw bending device.

In certain embodiments, the bending device is a ram bending device, the force die comprises a first counter die and a second counter die, and the bend die is a radius block.

In certain embodiments, the bending device is a three-roll bending device, the force die comprises a first counter roller and a second counter roller, and the bend die is a bend roller.

In certain embodiments, the bending device is a compression bending device, the force die comprises a stationary bend die, the bending device comprises a clamp, and the bend die is a compression die.

In certain embodiments, the bending device is a rotary draw bending device, the force die comprises a stationary pressure die, the bending device comprises a clamp, a follower slide, and a wiper die, and the bend die is a rotatable bend die.

In certain embodiments, the bending module is a passive bending module.

In certain embodiments, the bending device comprises an actuator for applying a force to the force die thereby causing a rod positioned between the force die and the bend die to bend around the bend die.

In certain embodiments, the force transfer device is arranged to transfer a force from an actuator to the force die.

In certain embodiments, the bending module is an active bending module.

In certain embodiments, the bending module comprises a cutter for cutting rods.

In certain embodiments, the bending module is at least in part a fixed module.

In certain embodiments, the bending module is arranged to be releasably connected to a mobile cart.

In certain embodiments, the bending module is a floating module.

In certain embodiments, the bending module is arranged to be releasably connected to a robotic arm.

In certain embodiments, the actuator is separate from the bending apparatus. In certain embodiments, the actuator is internal to a mobile cart

In certain embodiments, the bending apparatus is autoclavable. In certain embodiments, the bending apparatus is sterilizable.

In certain embodiments, the bending apparatus has at least one of a length, width, and height from 5.08 cm to 10.16 cm, 10.16 cm to 15.24 cm, 15.24 cm to 20.32 cm, 20.32 cm to 30.48 cm, and 30.48 cm to 60.96 cm.

In certain embodiments, the surgery is orthopedic surgery or spinal surgery.

In certain embodiments, the end-effector is configured to releasably hold the first surgical tool, allowing the first surgical tool to be removed and replaced with a second surgical tool.

In certain embodiments, the manipulator is configured to allow robotically assisted or unassisted positioning and/or movement of the end-effector by a user with at least six degrees of freedom, wherein the six degrees of freedom are three degrees of translations and three degrees of rotations.

In certain embodiments, the patient position is a position of one or more markers placed in spatial relation to one or more vertebrae.

In certain embodiments, controlling the actuator to move the end-effector comprises controlling the actuator to move the end-effector in a direction corresponding to a direction of application of the force and/or torque.

In certain embodiments, the end-effector is configured to move at a predetermined measured pace upon application and detection of user force and/or torque applied to the end-effector in excess of the predetermined minimum force and/or torque and the predetermined measured pace is a steady, slow velocity.

According to a further aspect it is provided a robotic surgical system for use in a surgical procedure, the system comprising: a robotic arm comprising an end-effector; an actuator for controlled movement of the robotic arm and positioning of the end effector; and a processor and a non-transitory computer readable medium storing instructions thereon wherein the instructions, when executed, cause the processor to:receive (e.g., determine) a desired curvature of a skeletal structure (e.g., spine, hip, leg, femur, tibia, fibia, hip, knee, or ankle) of a patient; determine a position of each the two or more screws in the patient during the surgical procedure (e.g., using a pointing device and a navigation system; e.g., wherein the position of each of the two or more screws is different than an ideal position of each screw); and intraoperatively determine the desired curvature of an implantable rod (e.g., for use in joining the heads of two or more screws) based at least in part on the desired curvature of the skeletal structure and the position of the two or more screws placed in the patient during the surgical procedure.

According to a further aspect it is provided a robotic surgical system for performing surgery, the system comprising: a robotic arm comprising an end-effector; an actuator for controlled movement of the robotic arm and positioning of the end effector; and a processor and a non-transitory computer readable medium storing instructions thereon wherein the instructions, when executed, cause the processor to: intraoperatively coordinate the bending of an implantable rod to produce a shaped rod based at least in part on a desired curvature of a skeletal structure and the position of each of two or more screws in the patient during the surgical procedure.

### BRIEF DESCRIPTION OF THE FIGURES

The foregoing and other objects, aspects, features, and advantages of the present disclosure will become more apparent and better understood by referring to the following description taken in conjunction with the accompanying drawings, in which:

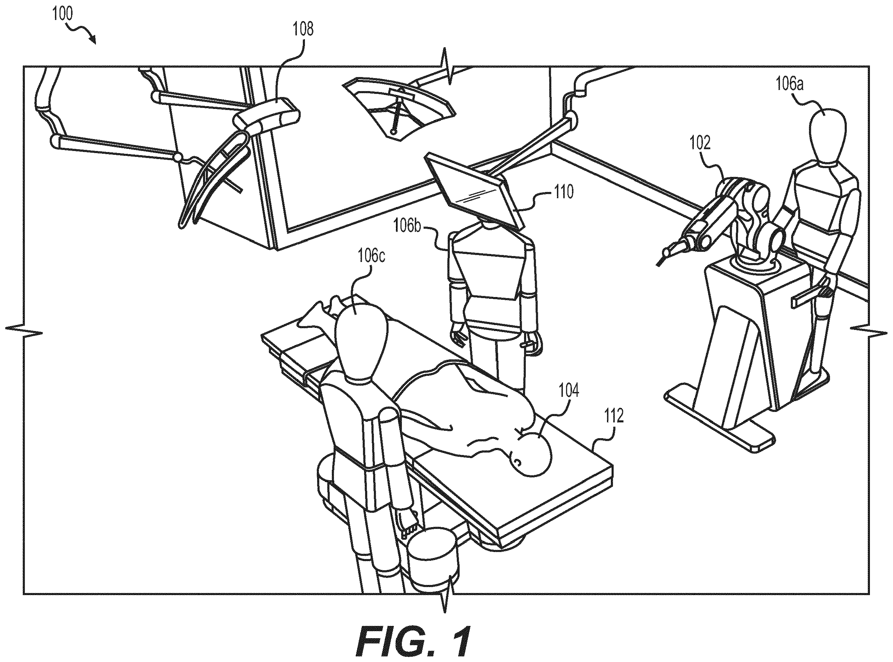

FIG. 1 is an illustration of an example robotic surgical system in an operating room;

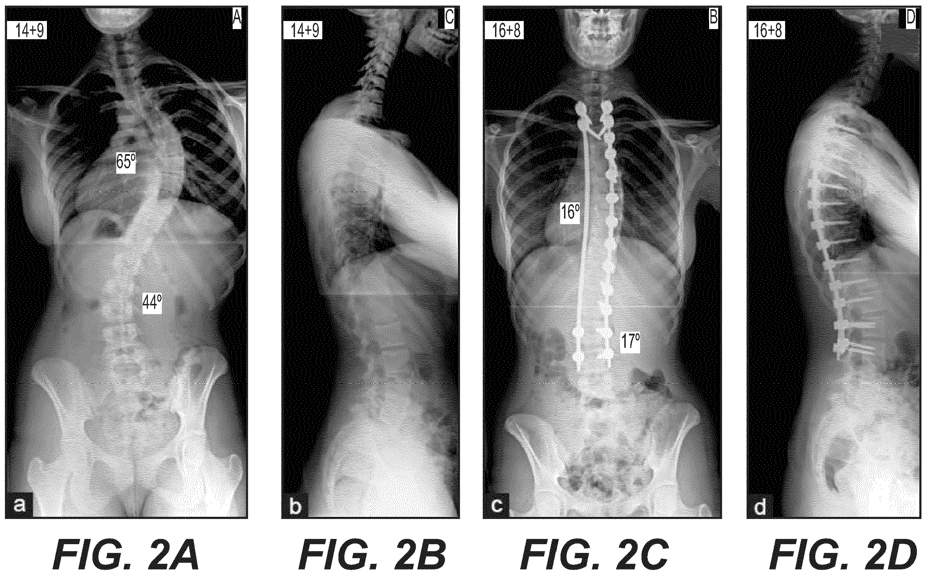

FIG. 2 is an illustration of how rods can be bent and implemented to correct the shape of a spine;

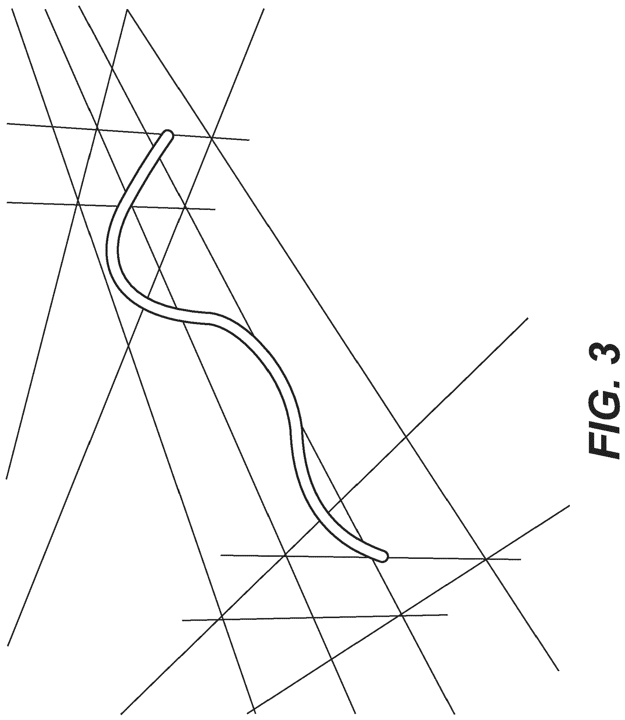

FIG. 3 is an illustration of a curvature that may be applied to a rod for use in correcting the shape of a spine;

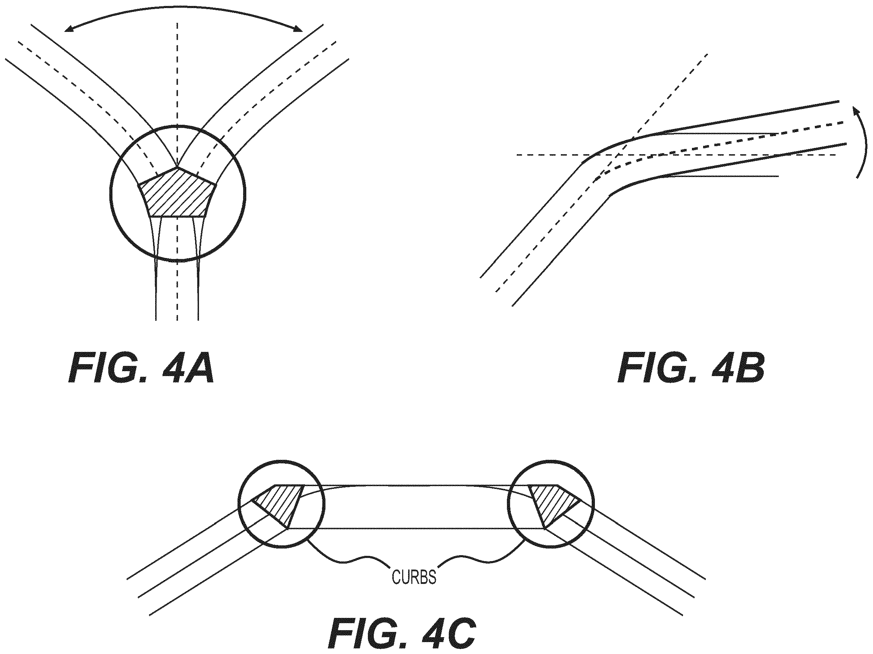

FIG. 4 is an illustration of the undesirable features that may occur when manually bending rods;

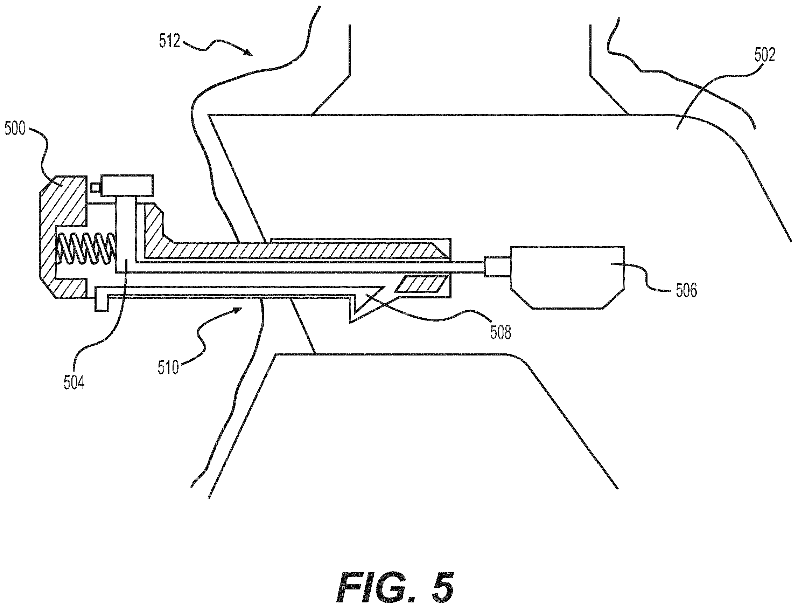

FIG. 5 is an illustration of a passive bending module;

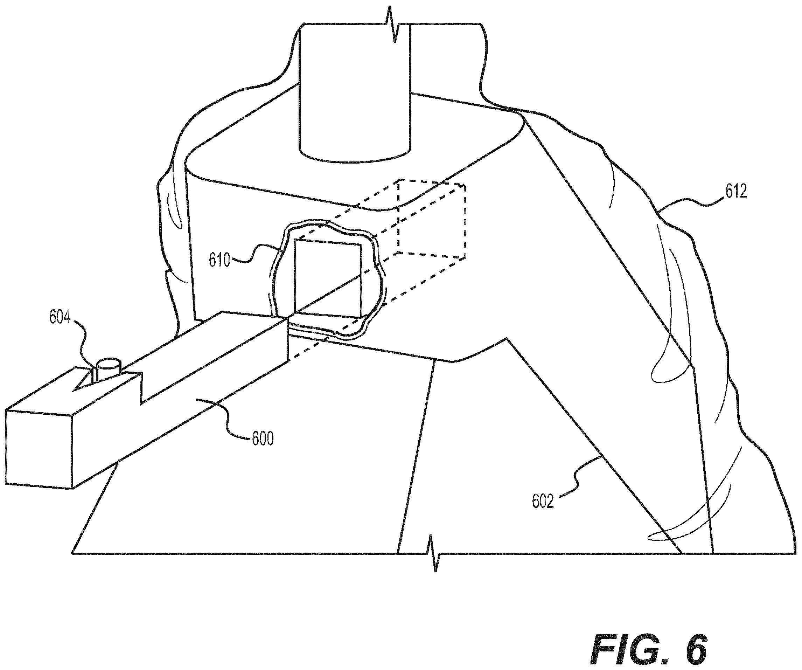

FIG. 6 is an illustration of a bending module before being inserted into a slot in the robotic system;

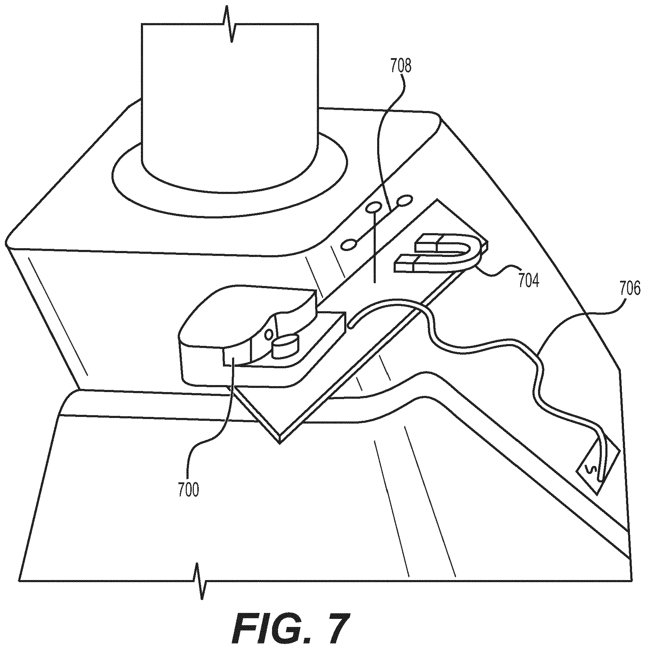

FIG. 7 is an illustration of an active bending module;

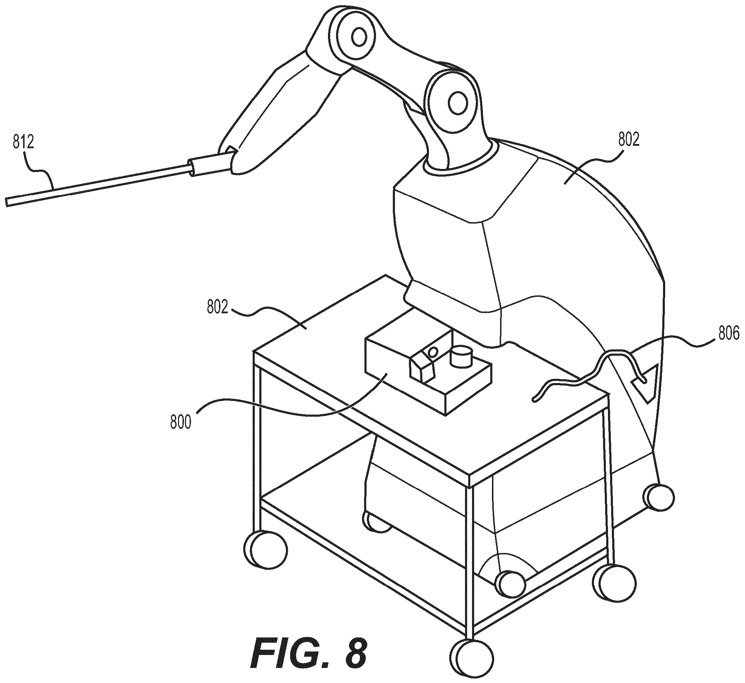

FIG. 8 is an illustration of a bending module on a cart;

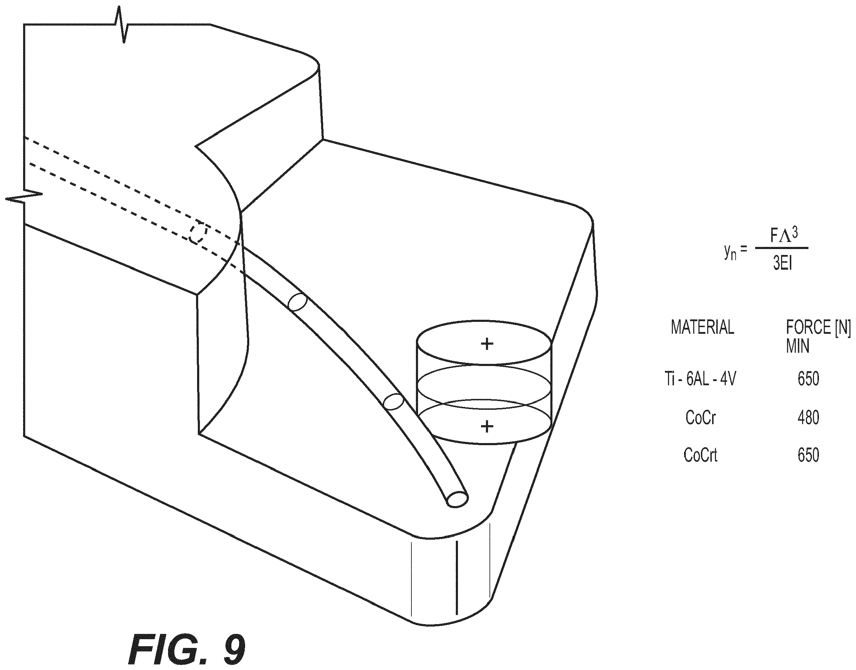

FIG. 9 is an illustration of an example analysis of the forces required to move a bending die of a bending module;

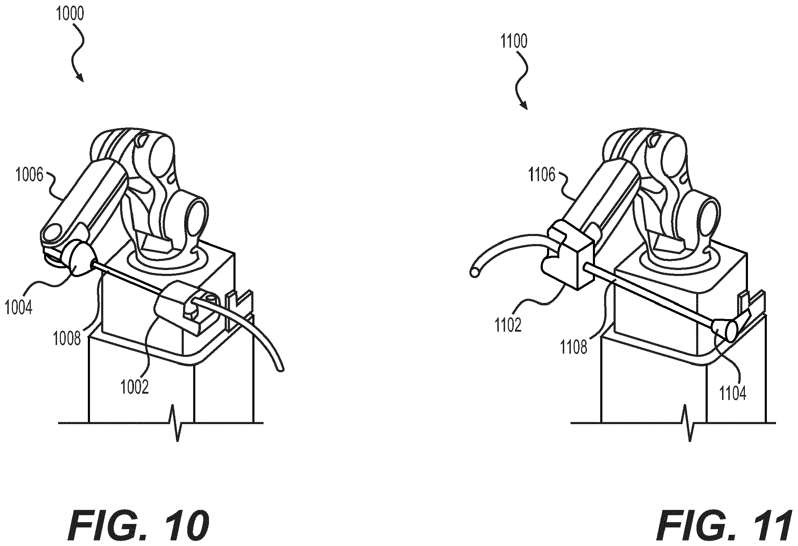

FIG. 10 is an illustrations of a fixed bending module;
FIG. 11 is an illustration, according to the invention, of a floating module;

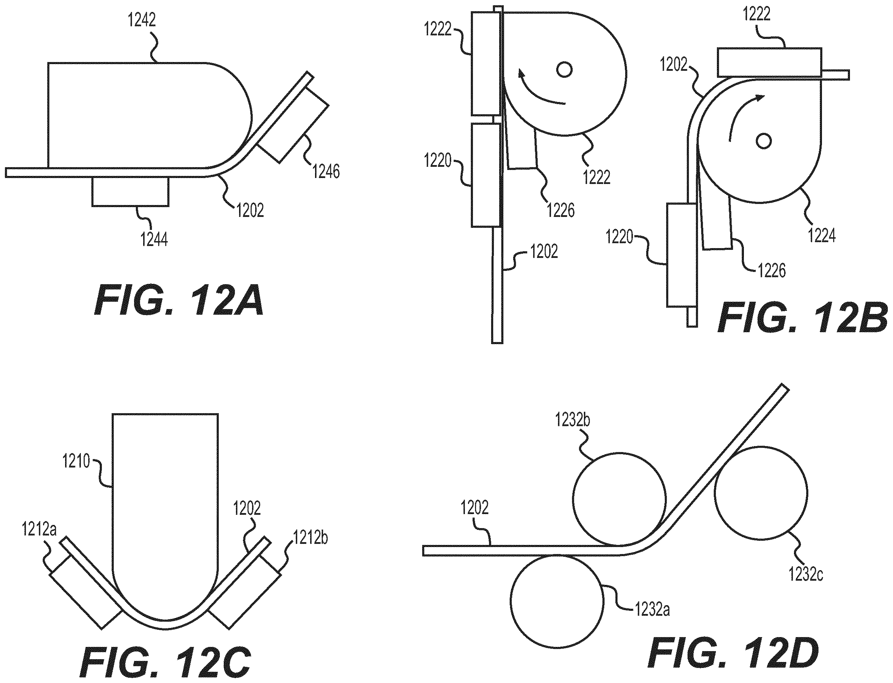

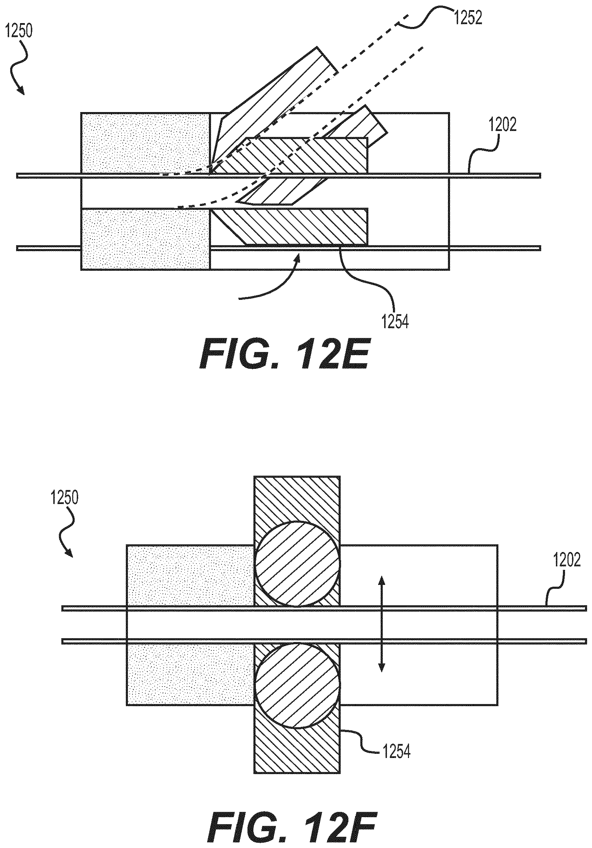

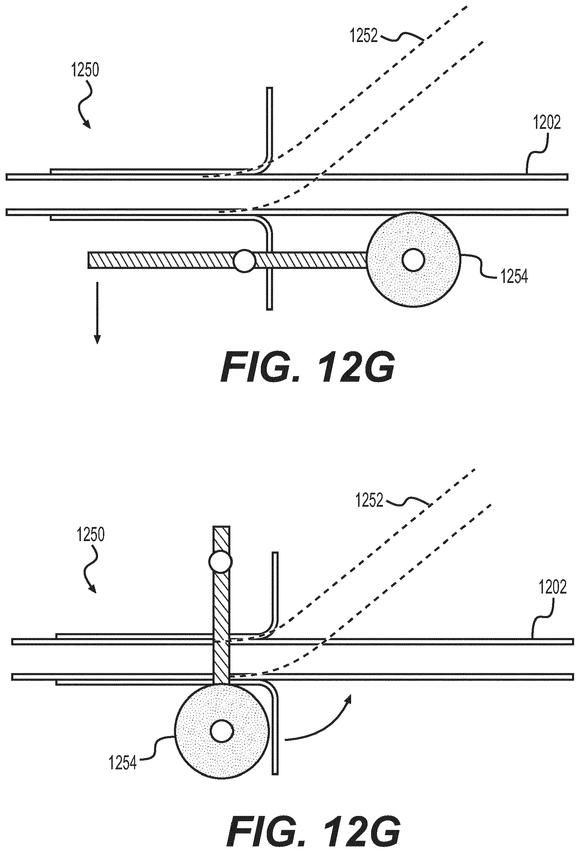

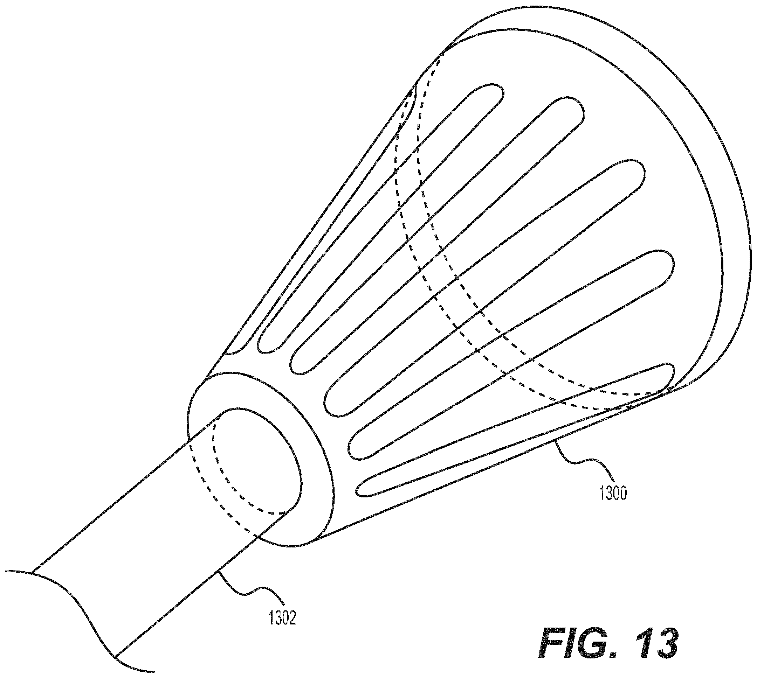

FIGS. 12A through 12H are illustrations of types of bending devices; FIG. 13 is an illustration of an example rod fixation apparatus;

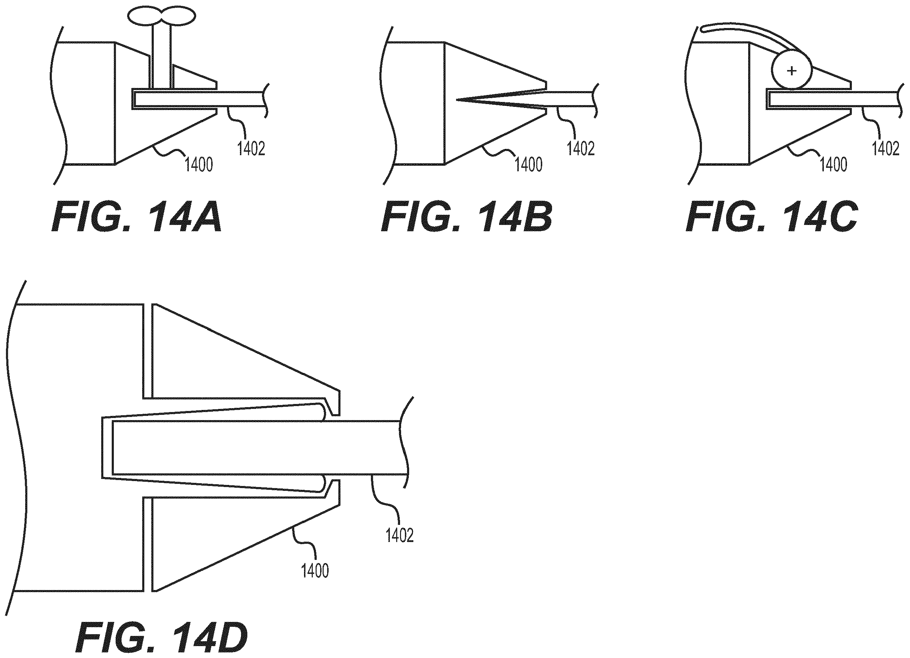

FIGS. 14A through 14D are illustrations of example fixation apparatuses; and

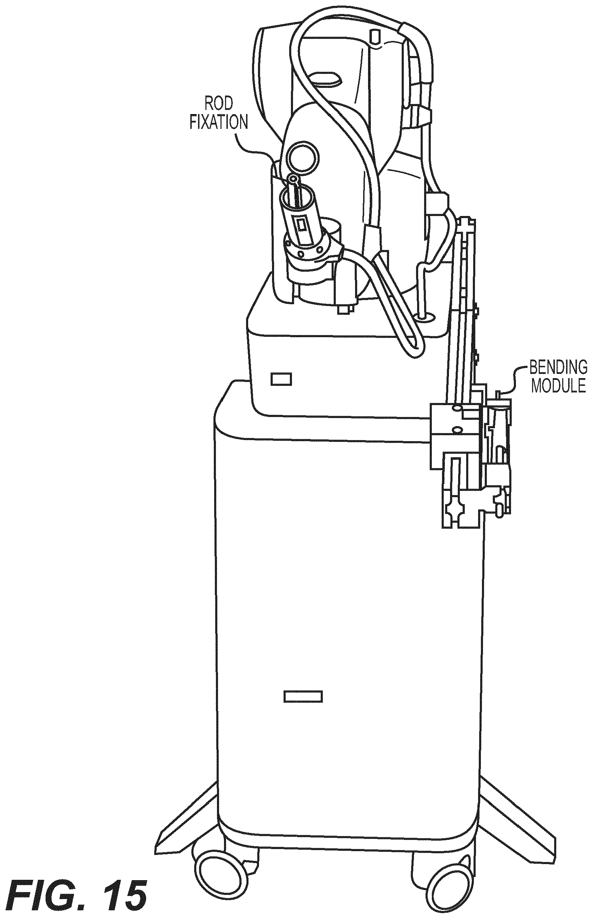

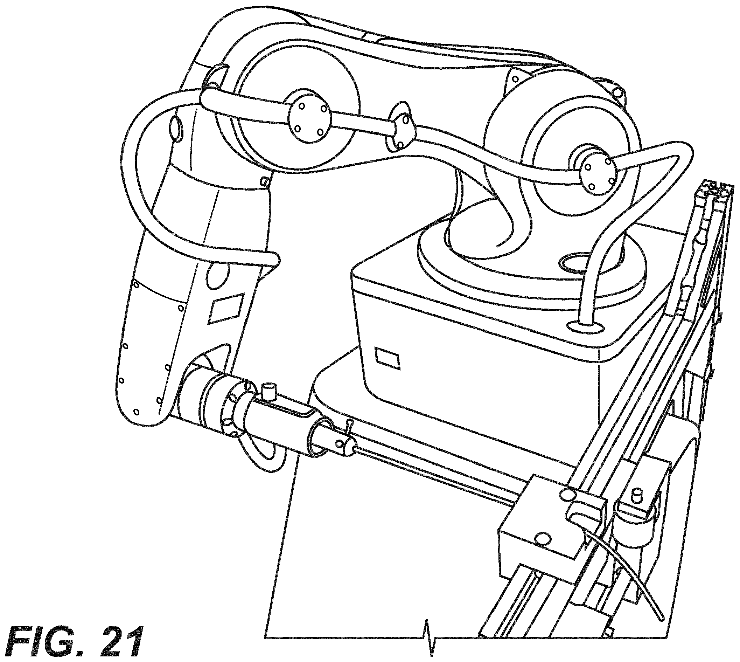

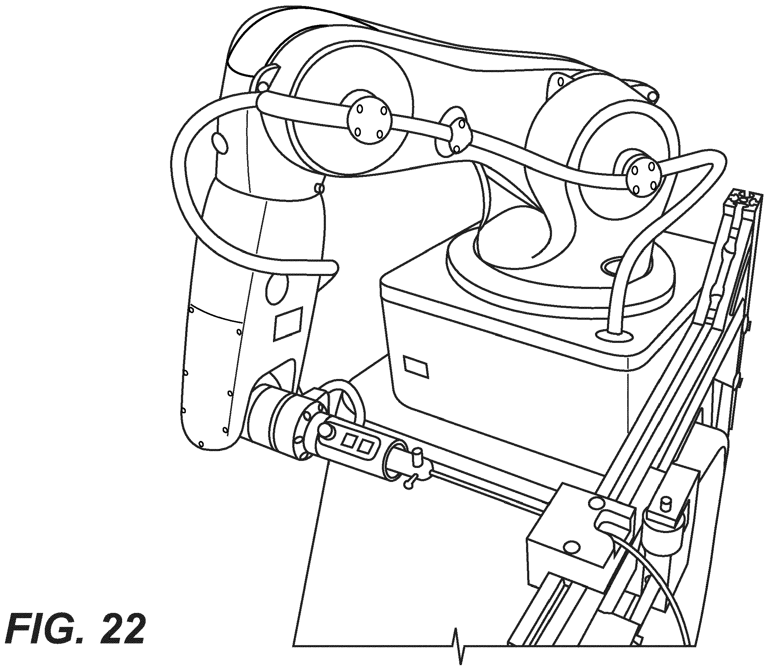

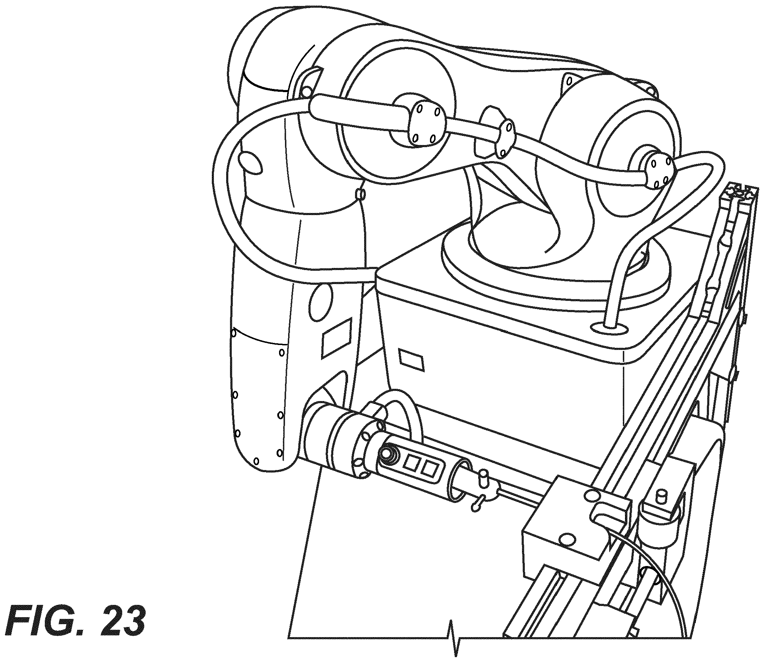

FIGS. 15 through 29 are photographs of an example robotic surgical system for bending rods.

The features and advantages of the present disclosure will become more apparent from the detailed description set forth below when taken in conjunction with the drawings, in which like reference characters identify corresponding elements throughout. In the drawings, like reference numbers generally indicate identical, functionally similar, and/or structurally similar elements.

### DETAILED DESCRIPTION OF THE INVENTION

FIG. 1 illustrates an example robotic surgical system in an operating room 100. In some implementations, one or more surgeons, surgical assistants, surgical technologists and/or other technicians (e.g., 106a-c) perform an operation on a patient 104 using a robotic-assisted surgical system. In the operating room 100 the surgeon may be guided by the robotic system to accurately execute an operation. This may be achieved by robotic guidance of the surgical tools, including ensuring the proper trajectory of the tool (e.g., drill or screw). In some implementations, the surgeon defines the trajectory intra-operatively with little or no preoperative planning. The system allows a surgeon to physically manipulate the tool holder to safely achieve proper alignment of the tool for performing crucial steps of the surgical procedure. Operation of the robot arm by the surgeon (or other operator) in force control mode permits movement of the tool in a measured, even manner that disregards accidental, minor movements of the surgeon. The surgeon moves the tool holder to achieve proper trajectory of the tool (e.g., a drill or screw) prior to operation or insertion of the tool into the patient 104. Once the robotic arm is in the desired position, the arm is fixed to maintain the desired trajectory. The tool holder serves as a stable, secure guide through which a tool may be moved through or slid at an accurate angle. Thus, the disclosed technology provides the surgeon with reliable instruments and techniques to successfully perform his/her surgery.

In some embodiments, the operation may be spinal surgery, such as a discectomy, a foraminotomy, a laminectomy, or a spinal fusion, or orthopedic surgery, such as knee, shoulder, hip, leg, or ankle surgery. In some implementations, the surgical robotic system includes a surgical robot 102 on a mobile cart 114. The surgical robot 102 in the example shown in FIG. 1 is positioned in proximity to an operating table 112 without being attached to the operating table 112, thereby providing maximum operating area and mobility to surgeons around the operating table 112 and reducing clutter on the operating table 112. In alternative embodiments, the surgical robot 102 (or cart) is securable to the operating table 112. In certain embodiments, both the operating table 112 and the cart 114 are secured to a common base to prevent any movement of the cart or table 112 in relation to each other, even in the event of an earth tremor.

The mobile cart 114 may permit a user (operator) 106a, such as a technician, nurse, surgeon, or any other medical personnel in the operating room 100, to move the surgical robot 102 to different locations before, during, and/or after a surgical procedure. The mobile cart 104 enables the surgical robot 102 to be easily transported into and out of the operating room 100. For example, a user 106a may move the surgical robot 102 into the operating room 100 from a storage location. In some implementations, the mobile cart 114 may include wheels, a track system, such as a continuous track propulsion system, or other similar mobility systems for translocation of the cart. The mobile cart 114 may include an attached or embedded handle for locomotion of the mobile cart 114 by an operator (e.g., user 106a).

For safety reasons, the mobile cart 114 may be provided with a stabilization system that may be used during a surgical procedure performed with a surgical robot 102. The stabilization device increases the global stiffness of the mobile cart 114 relative to the floor in order to ensure the accuracy of the surgical procedure. In some implementations, the wheels include a locking device that prevents the cart 114 from moving. The stabilizing, braking, and/or locking device may be activated when the machine is turned on. In some implementations, the mobile cart 114 includes multiple stabilizing, braking, and/or locking devices. In some implementations, the stabilizing device is electro-mechanical with electronic activation. The stabilizing, braking, and/or locking device(s) may be entirely mechanical. The stabilizing, braking, and/or locking device(s) may be electronically activated and deactivated.

In some implementations, the surgical robot 102 includes a robotic arm mounted on a mobile cart 114. An actuator may move the robotic arm. The robotic arm may include a force control end-effector configured to hold a surgical tool. The robot 102 may be configured to control and/or allow positioning and/or movement of the end-effector with at least four degrees of freedom (e.g., six degrees of freedom, three translations and three rotations).

In some implementations, the robotic arm is configured to releasably hold a surgical tool, allowing the surgical tool to be removed and replaced with a second surgical tool. The system may allow the surgical tools to be swapped without re-registration, or with automatic or semiautomatic re-registration of the position of the end-effector.

In some implementations, the surgical system includes a surgical robot 102, a tracking detector 108 (e.g., navigation system) that captures the position of the patient and different components of the surgical robot 102, and a display screen 110 that displays, for example, real time patient data and/or real time surgical robot trajectories.

In some implementations, a tracking detector 108 monitors the location of patient 104 and the surgical robot 102. The tracking detector 108 may be a camera, a video camera, an infrared detector, field generator and sensors for electro-magnetic tracking or any other motion detecting apparatus. In some implementation, based on the patient and robot position, the display screen 110 displays a projected trajectory and/or a proposed trajectory for the robotic arm of robot 102 from its current location to a patient operation site. By continuously monitoring the patient 104 and robotic arm positions, using tracking detector 108, the surgical system can calculate updated trajectories and visually display these trajectories on display screen 110 to inform and guide surgeons and/or technicians in the operating room 100 using the surgical robot. In addition, in certain embodiments, the surgical robot 102 may also change its position and automatically position itself based on trajectories calculated from the real time patient and robotic arm positions captured using the tracking detector 108. For instance, the trajectory of the end-effector can be automatically adjusted in real time to account for movement of the vertebrae and/or other part of the patient 104 during the surgical procedure. An example robotic surgical system that may be used with the disclosed technology or modified for use with the disclosed technology is described in U.S. Patent Application No. 14/266769, filed April 30, 2014, published as US 2015/0100066 A1, and entitled Apparatus, Systems, and Methods for Precise Guidance of Surgical Tools.

FIG. 5 is an illustration of a passive bending module 500, for example, without actuators therein (e.g., no active parts). Specifically, FIG. 5 illustrates a cross-cut of a portion of a robotic surgical system 502 and a bending module 500. The bending module 500 is a passive device having no active parts (e.g. actuators). This allows the device to be easily sterilized in an autoclave. The module contains a bending element 504 (i.e., force transfer device) which is moved by a linear actuator 506. The module is fixed partially inside robot (e.g., inside a mobile cart of the robotic surgical system). A quick fixation apparatus automatically fixes itself in the desired position after insertion and requires a deliberate action to be removed. In this example, the linear actuator is inside the mobile cart of the robot 502. Thus, in certain embodiments, the linear actuator 506 does not need to be sterile. A sterile drape 512 can include a passageway 510 to allow the bending module 500 to extend therethrough to separate the sterile environment from the non-sterile environment. An example of a sterile drape 512 and the types of drape passages 510 that can be used herein to allow portions of the bending module 500 to be inside the sterile environment and outside the sterile environment are described in described in U.S. Patent Application No. 14/602,627, filed July 27, 2015, published as US2015/0202009 A1, and entitled "Sterile Drape and Adapter for Covering a Robotic Surgical Arm and Preventing Contamination of a Sterile Field".

FIG. 6 illustrates the bending module 600 right before being inserted into slot in the robotic system 602. In certain embodiments, the disclosed technology includes a sterile drape 612 with a sleeve or hole 610 which allows for passing the bending module 600 through the drape 612 so that it can securely couple to the robot 602. In certain embodiments, the sleeve 610 of the

drape 612 is sealed using sterile tape (e.g., such that the sleeve is secured tightly around a portion of the bending module). The tape may be wrapped around the sleeve 610 to seal the sleeve 610. In some implementations, the sleeve 610 is part of the sterile drape 612. In some implementations, the sleeve 610 is separate from the drape 612 and the interface between the sleeve 610 and the sterile drape 612 is sealed using sterile adhesive tape or another sterile adhesive material.

In certain embodiments, a sterile adapter is used to secure the drape to the robot around the opening in the drape that allows the bending module to be connected to the robot.

The sterile adapter may be a disposable (e.g. a single-use product). For example, a new sterile adapter may be used for every surgical procedure. In some implementations, the sterile adapter is a rigid or semi-rigid device. It may be made from a hard plastic, polymer, or a composite material. In some implementations, the sterile adapter secures a drape over a surgical robot to prevent contamination of a sterile field.

The sterile adapter may include a rigid or semi-rigid collar (e.g., ring or a hollow cylindrical structure) configured to mount (e.g., snap-mount) onto an interface of the surgical robotic arm. The sterile adapter may include a rigid or semi-rigid body extending from the collar and shaped to conform to a portion of the surgical robotic arm to tightly secure a flexible drape in place (e.g., with no folds) over the portion of the surgical robotic arm when the drape is attached to the adapter.

In some implementations, the body is one or more tabs (e.g., 3, 4, 5, 6, 7, or 8 tabs) that engage an interface on the robot. The tabs may "click" into the interface to provide easy and secure mounting of the sterile adapter, and hence sterile drape, on the robot. The sterile drape may be glued or welded to the sterile adapter (e.g., during manufacturing). The adapter 200 ensures that the drape is tightly stretched over the tool holder and robot interface to provide repeatable and rigid positioning of the tool holder relative to the robotic arm. The sterile drape can be coupled, via glue or welding, to a sterile adapter. In some implementations, the sterile drape is glued or welded to the sterile adapter. After the welding/gluing dries the part of the drape inside the sterile adapter is stretched. The sterile drape can be tightly stretched over the opening of the sterile adapter (e.g., the opening
through which the bending module passes to connected to the robot). When the sterile adapter is attached to the robot (e.g., clicked into the interface on the robot), the robot will be covered by the sterile drape that is stretched over the opening of the sterile adapter. As described below, in some implementations, positioning elements and a tightening screw will protrude through the opening of the sterile adapter and piece the sterile drape when the tool support is applied to the robotic arm.

FIG. 7 is an illustration of an active bending module 700. Sterility of the active bending module 700 can be achieved by using drapes and sterilizable elements. Additionally, a sterilizable electric motor can be used, such as the EC 19 Brushless DC Motor from Maxon Motor AG of Sachseln, Switzerland.

An active bending module 700 has actuators built in. It is fixed to the robot 702 using a fixation apparatus 704. The fixation apparatus 704 can be magnetic, electro-magnetic, mechanical (e.g. using lever device). In certain embodiments, there is no need for a strong fixation between the robot 702 (e.g., mobile cart) and the bending module 700. During bending most of the large forces and reactions happen within the bending module and do not transmit to the fixation. The positioning of the bending module in reference to the robotic system shall be known by the surgical system (e.g., the robotic surgical system's computer and/or navigation system). This can be achieved by placing the module 700 in a known, pre- determined position, relative to the cart (e.g. using positioning pins, rails, etc.) and/or identifying the position of the module 700 using a navigation marker 708 or by driving robotic arm manually to one or more known points on the bending module 700 to register the position of the bending module 700.

The bending module 700 can be connected to the cart of the robot 702 using, in certain embodiments, a connection cable. In certain embodiments, a mechanical connection is used to actuate the bending module 700. The connection cable 706 can provide power to bending module actuators, actuate them directly, or just send relevant data to bending module internal electronics/logic. In certain embodiments, the movement of the bending element inside bending module 700 is synchronized with movement of the robotic arm.

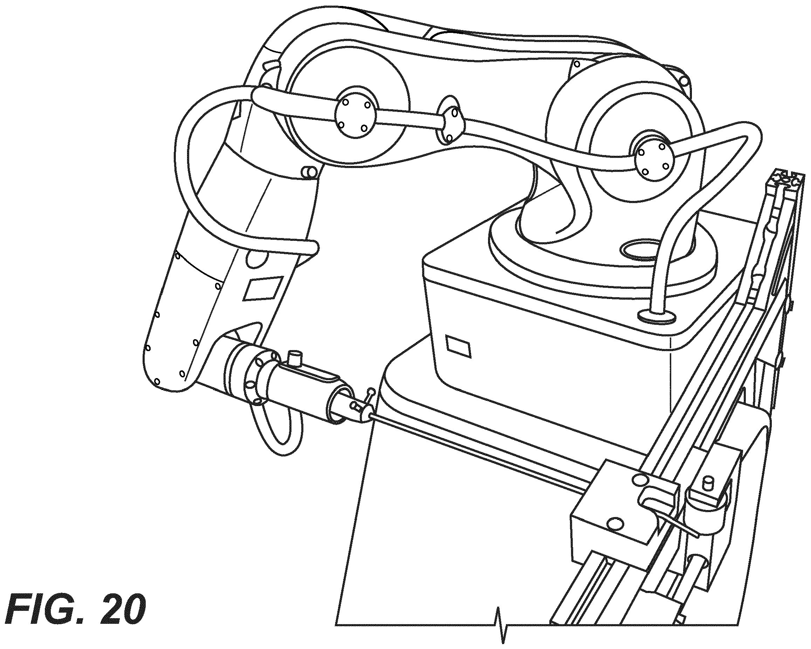

Alternatively, as shown in FIG. 8, the bending module 800 can be placed on the separate cart 802 as shown in the Figure above. The cart can be fixed to the robot 802 (e.g., mobile cart of the robot 802) using a fixation apparatus as described above. The precise position of the bending module 800 (navigation, robot registration) can be determine similar manners as described above regardless of the location of the bending module 800. Bending module 800 on the cart uses connection cable 806 for the similar purpose. As shown in FIG. 20 8, a rod 812 can be held by the robot 802 and inserted in the bending module 800 by the robot 802 so the rod 812 can be bent.

FIG. 9 is an illustration of an example analysis of the forces required to move a bending die of a bending module a particular length depending on the material of the rod.

FIG. 10 is an illustration of a fixed bending module 1002. In this example the bending module 1002 is fixed and/or at least partially integrated directly into the robot 1000 (e.g., mobile cart). A rod fixation 1004 is attached to the robotic arm 1006 to grasp the rod 1008 such that the robotic arm 1006 can move the rod 1008 and precisely control the positioning of the rod 1008 within the bending module 1002.

FIG. 11 is an illustration, according to the invention, of a floating bending module 1102. The bending module 1102 is coupled to the robotic arm 1106 and the rod fixation 1104 is connected or integral to the robot 1100 (e.g., mobile cart). The robotic arm 1106 positions the bending module 1102 along the rod 1108 in the appropriate places and the bending module 1102 is activated in these locations to bend the rod 1108 to the appropriate shape.

FIGS. 12A through 12H illustrate various types of bending devices. For example, the bending device used in the bending module may be a compression bending device, a rotary draw bending device, a ram bending device , or a three-roll bending device as shown in FIGS. 12A through 12D respectively.

As shown in FIG. 12A, compression bending utilizes a fixed bending die 1242 and a clamp 1244 and 1246 on each end of the rod 1202. One of the clamps (e.g., 1246) is moved thereby forcing the rod 1202 to bend around the bending die 1242 (e.g., up to 180 degrees).

In certain embodiments, a compression bending module can bend, twist, and lift an extrusion simultaneously to create unique shapes.

As shown in FIG. 12B, rotary draw bending utilizes a stationary or sliding pressure die 1220 and clamping block 1222 to hold a rod 1202 in place. A round bending die 1224 is rotated (e.g., up to 90 degrees) thereby bending the rod 1202 as it rotates. Using this method a rod 1202 can only be bent one radius at a time. The round bending die 1224 can be powered by hydraulics. A mandrel or other component can be incorporated to grip the rotary die 1224 such that creasing or misshaping of the rod 1202 is prevented. A wiper die 1226 is angled slightly off parallel from the workpiece to ensure the die's edge makes full contact with the tube just before the tangent point of the tube's inner radius.

As shown in FIG. 12C, ram bending uses a ram 1210 to force the rod 1202 on a pressure/bending dies 1212a-b. A ramming die 1210 pushes the rod 1202 onto the pressure dies 1212a-b, forcing the rod 1202 into the desired bended form

A shown in FIG. 12D, three-roll bending pushes the rod 1202 around three different rolls 1232a-c placed in a triangular shape. The rolls 1232a-c are adjusted to form a precise angle, up to a 360-degree rotation, that can roll horizontally or vertically. The rod 1202 curves and bends as it is moved across the rollers 1232a-c. In certain embodiments, two of the rollers (e.g., 1232a and 1232c) are stationary and the third roller (e.g., 1232b) is a power roller that can be moved to change the bend radius.

FIGS. 12E through 12H illustrate additional bending modules. The rod 1202 is shown extending through the bending module 1250 prior to bending and the bent portion is shown after bending and labeled as 1252. The force dies 1254 are used to apply a force to the rod thereby bending the rod. The arrows show the direction of the forces that can be applied in the examples shown.

FIG. 13 is an illustration of an example rod fixation apparatus 1300. The rod fixation apparatus 1300, in certain embodiments, is used to securely hold the rod 1302. The rod fixation apparatus 1300 can be attached to the robot and integrated into the robot. In certain embodiments, the rod fixation apparatus 1300 is releasably secured to the mobile cart. In other embodiments, the rod fixation apparatus 1300 is releasably secured to the robotic arm.

The arm can move the rod fixation apparatus 1300 and hence the rod 1302 secured therein to the appropriate positions such that the rod 1302 is bent in the desired configuration. This allows the bending device to be a simple device that simple bends a rod 1302. The determination of where and how to bend the rod 1302 can be done outside of the bending module itself and the robotic arm can position the rod 1302 and activate the bending module to produce the appropriately shaped rod 1302. FIGS. 14A though 14D illustrate example rod fixation apparatuses 1400 and specifically the device by which the rod fixation apparatus 1400 securely holds the rod 1402.

FIG. 15 is a photograph of an example robotic surgical system with a bending module. As shown in this photograph, the rod fixation is attached to the robotic arm and the bending module is attached to the mobile car. Other orientation and/or connection points are available as well. For example, the rod fixation may be secured to the mobile cart and the bending module may be secured to the robotic arm.

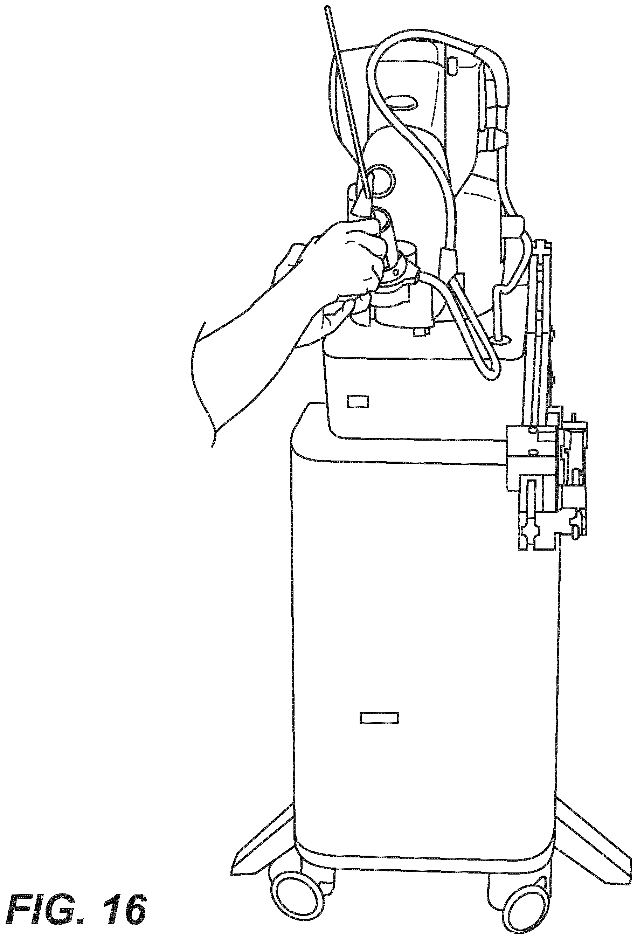

FIG. 16 is a photograph of a rod being inserted touch-free (e.g., without being touched by the user's hands) into the rod fixation on the robotic arm. In this example, the rod is inserted into the rod fixation using pliers.

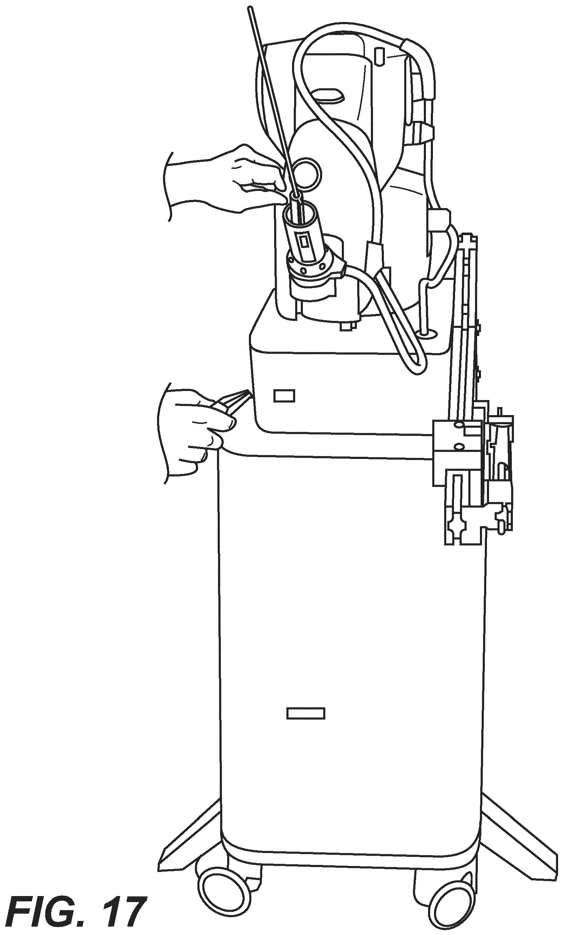

After inserting the rod into the rod fixation, the rod fixation is tightened to secure the rod therein as shown in FIG. 17. In this example, two bolts are tightened to secure the rod therein. In other examples more or less bolts are used or other grasping devices are used.

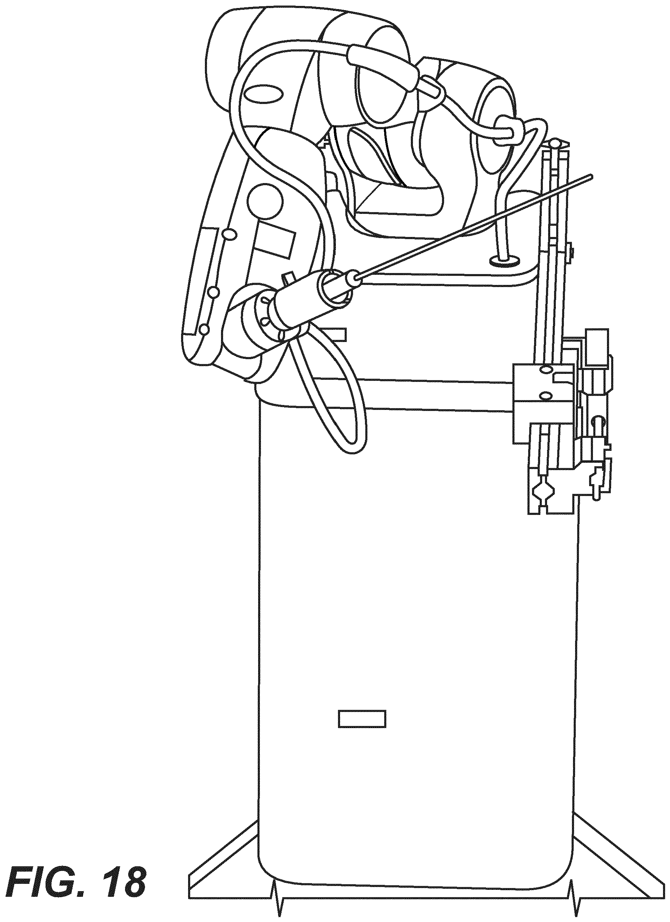

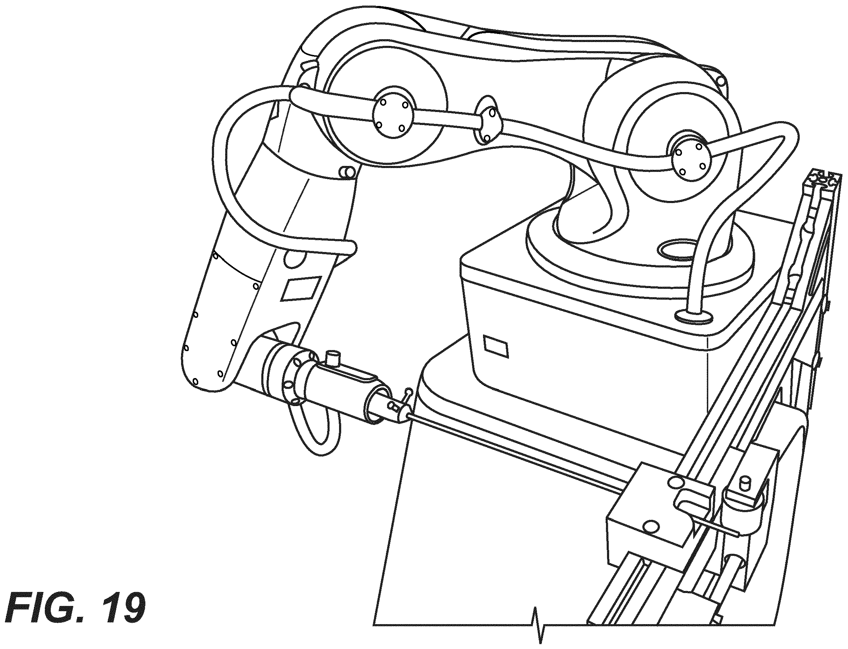

FIG. 18 is a photograph of the robotic surgical system moving the rod into the bending module so that it can be bent. In FIG. 19 the robotic surgical system has inserted the rod into the bending module and is being advanced to the appropriate location so it can be bent. FIG. 20 is a photograph of the bending module bending the rod. The robotic surgical system advances the rod, bends the rod, and repeats this process as shown in FIGS. 20 through 23 until the desired shaped rod is created. For example, the robotic arm advances the rod a 20 determined distance into the bending module, the bending modules bends the rod, and then the robotic arm advances the rod a second determined distance. This continues until the desired shaped rod is create. Cutting of the rod by the bending module can be automatic.

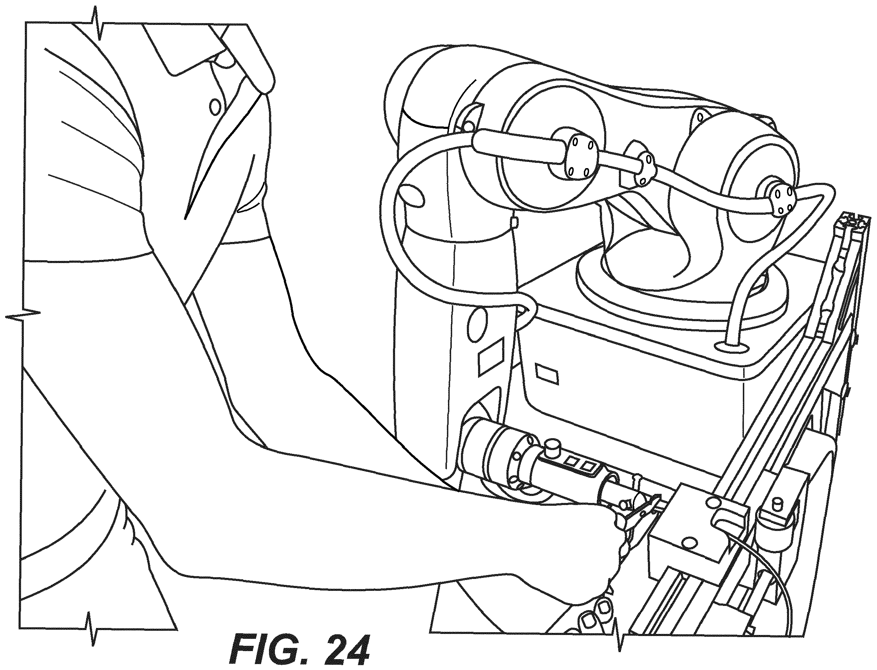

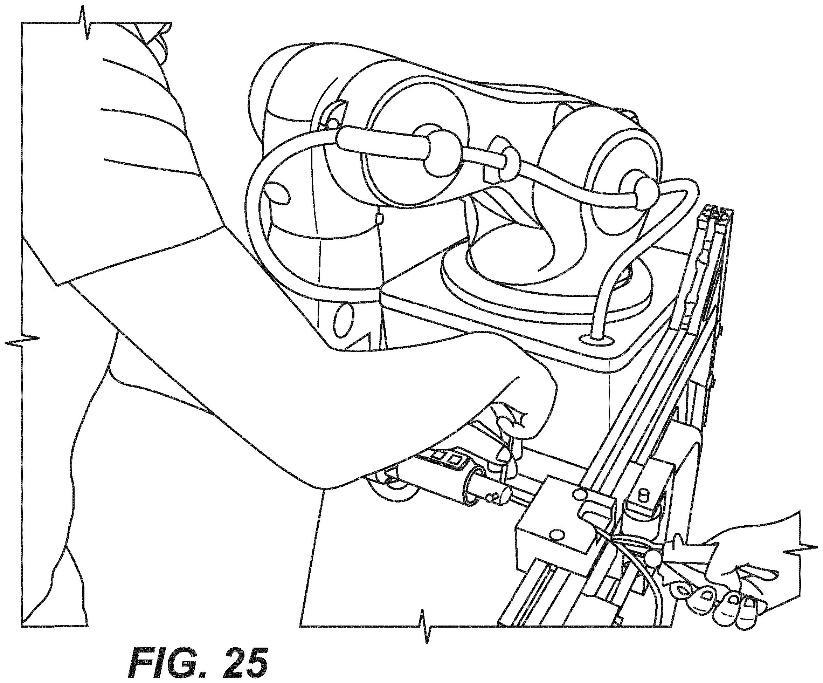

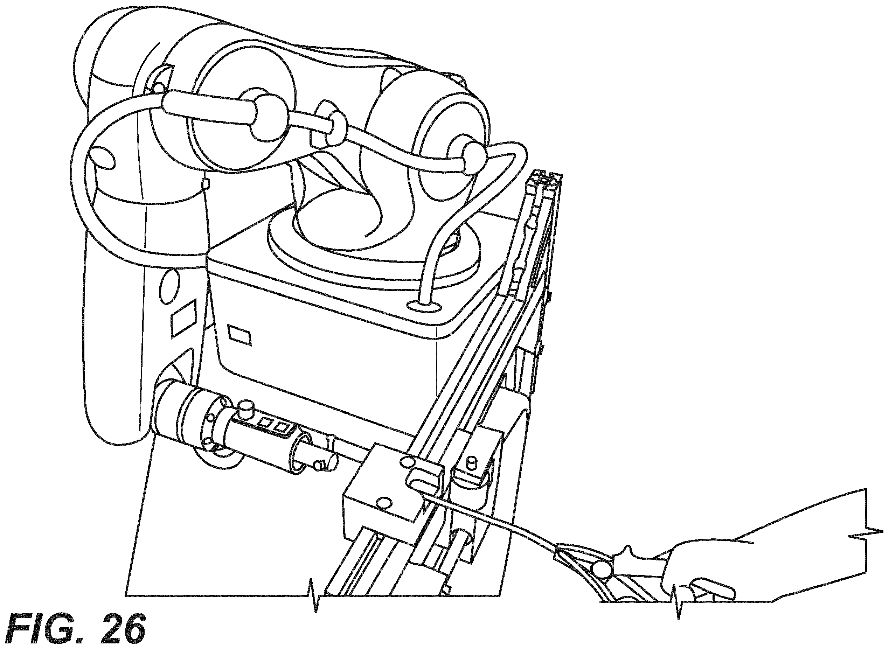

The rod is cut after the rod is bent into the desired shape. As shown in FIG. 24 the rod can be cut manually. In certain embodiments, a cutting device is integrated into the bending modules. In some implementations, instead of cutting the rod the rod fixation is loosened as shown in FIG. 25 such that the rod can be removed. After the rod is cut or the rod fixation is loosened, the rod can be removed as shown in FIG. 26.

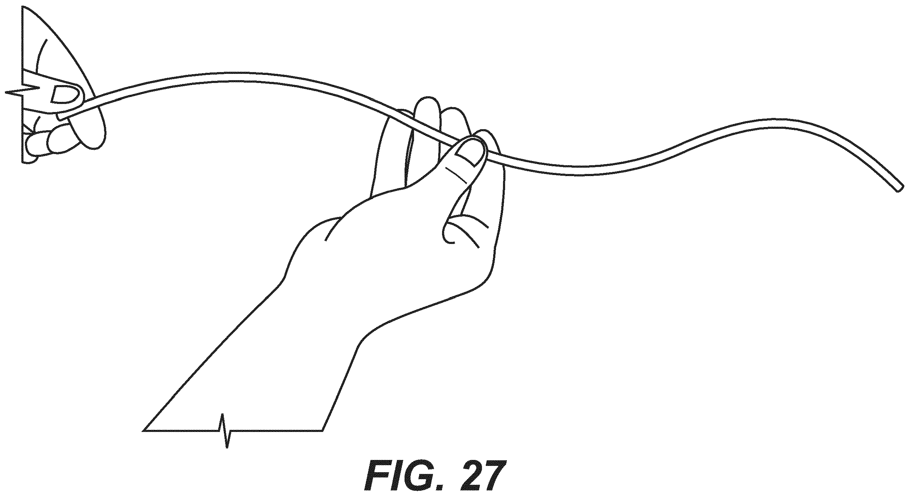

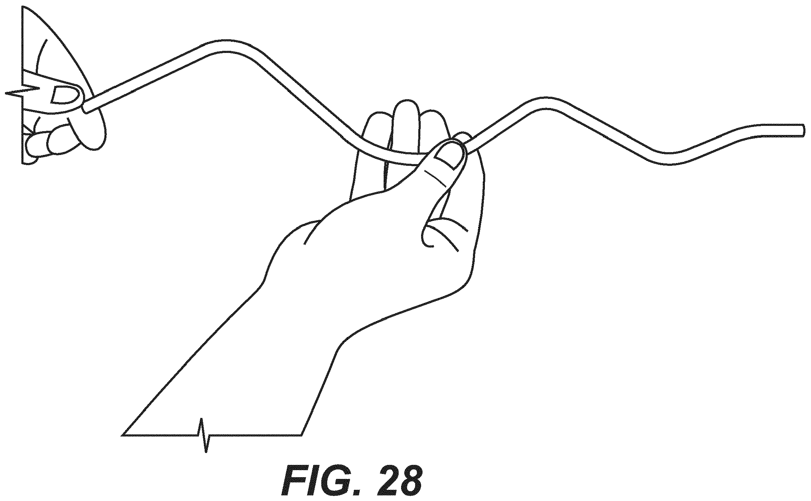

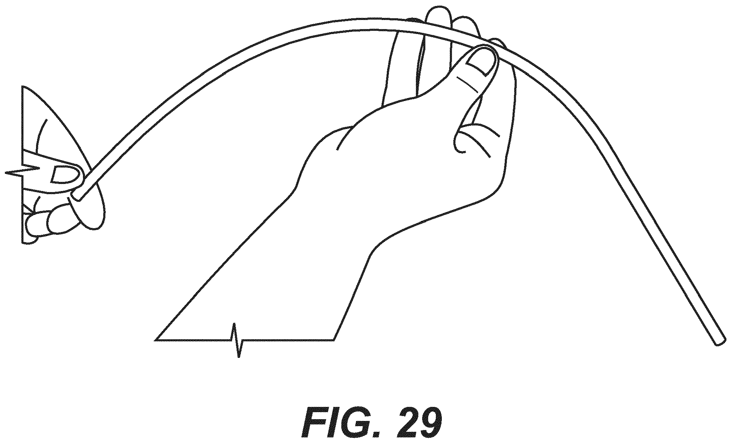

FIG. 27 is a photograph of a rod bent using the disclosed technology to provide smooth bends. FIG. 28 is an illustration of a rod bent using the disclosed technology. As shown in FIG. 28, the disclosed technology can be used to provide rods bent with a small bend radius even in tough materials such as CoCr+. FIG. 29 is an illustration of a rod bent using the disclosed technology to produce a rod with a three-dimensional curve.

In view of the structure, functions and apparatus of the systems and methods described here, in some implementations, a system and method for performing surgery with a robotic surgical system are provided. Having described certain implementations of methods and apparatus for supporting a robotic surgical system, it will now become apparent to one of skill in the art that other implementations incorporating the concepts of the disclosure may be used.

## Claims

1. A machine for intraoperative bending of surgical rods, the machine comprising:
a bending apparatus (500, 700) for bending a surgical rod (1202), the bending apparatus (500, 700) comprising:
a force die (1254);
a bend die (1250); and
a force transfer device (504) that transfers energy from an actuator (506) to the force die (504) thereby causing a surgical rod (1202) positioned between the force die (504) and the bend die (1250) to bend around the bend die (1250); and **characterized in that** it further comprises
a fixation apparatus (704) configured to releasably secure the bending apparatus (500,700) to a portion of a robotic surgical system that includes a robot arm and a robot base,
wherein the bending apparatus (500, 700) is arranged to be releasably connected to the robot arm.

2. The machine of claim 1, wherein the fixation apparatus (704) is magnetic, electromagnetic, and/or mechanical (e.g. using lever device).

3. The machine of claim 1 or 2, wherein the fixation apparatus (704) comprises at least one of push clips, push in rivets, screw rivets, clips, and tabs configured to attach to an interface on the robotic surgical system.

4. The machine of claim 1 or 2, wherein the fixation apparatus (704) comprises one or more holes sized for one or more bolts to pass through to secure the bending apparatus (500, 700) to the robotic surgical system via the one or more bolts.

5. The machine of any one of claims 1 to 4, wherein the bending apparatus (500,700) is a ram bending apparatus (1210), three-roll bending apparatus (1232), compression bending apparatus (1242), or rotary draw bending apparatus (1220).

6. The machine of any one of claims 1 to 4, wherein the bending apparatus (500, 700) is a ram bending apparatus (1210), the force die comprises a first counter die and a second counter die, and the bend die is a radius block.

7. The machine of any one of claims 1 to 4, wherein the bending apparatus is a three-roll bending apparatus (1232), the force die comprises a first counter roller and a second counter roller, and the bend die is a bend roller.

8. The machine of any one of claims 1 to 4, wherein the bending apparatus is a compression bending apparatus (1242), the force die comprises a stationary bend die, the bending apparatus comprises a clamp, and the bend die is a compression die.

9. The machine of any one of claims 1 to 4, wherein the bending apparatus is a rotary draw bending apparatus (1220), the force die comprises a stationary pressure die, the bending apparatus comprises a clamp, a follower slide, and a wiper die, and the bend die is a rotatable bend die.

10. The machine of claim 1, wherein the bending apparatus is a passive bending apparatus (500), wherein the passive bending apparatus (500) operates without using an actuator.

11. The machine of claim 1, wherein the bending apparatus (700) comprises an actuator for applying a force to the force die thereby causing a rod positioned between the force die and the bend die to bend around the bend die.

12. The machine of claim 11, wherein the force transfer device (504) is arranged to transfer a force from an actuator (506) to the force die (504).

13. The machine of claim 1, wherein the bending apparatus (700) is an active bending apparatus (700), wherein the active bending apparatus (700) comprises one or more actuators.

14. The machine of any one of the preceding claims, wherein the bending apparatus comprises a cutter for cutting rods.

15. The machine of claim 1, wherein the bending apparatus (500, 700) is at least in part a fixed module (1002).

## Patentansprüche

1. Maschine zum intraoperativen Biegen von chirurgischen Stangen, wobei die Maschine aufweist:
eine Biegevorrichtung (500, 700) zum Biegen einer chirurgischen Stange (1202), wobei die Biegevorrichtung (500, 700) umfasst:
einen Kraftstempel (1254);
ein Biegewerkzeug (1250); und
eine Kraftübertragungsvorrichtung (504), die Energie von einem Aktuator (506) zum Kraftstempel (504) überträgt, wodurch eine zwischen dem Kraftstempel (504) und dem Biegewerkzeug (1250) positionierte chirurgische Stange (1202) dazu gebracht wird, sich um das Biegewerkzeug (1250) zu biegen; und **dadurch gekennzeichnet, dass** sie ferner Folgendes aufweist
eine Fixierungsvorrichtung (704), die dazu ausgelegt ist, die Biegevorrichtung (500, 700) lösbar an einem Teil eines robotischen chirurgischen Systems zu fixieren, das einen Roboterarm und eine Roboterbasis umfasst,
wobei die Biegevorrichtung (500, 700) dazu vorgesehen ist, lösbar mit dem Roboterarm verbunden zu sein.

2. Maschine nach Anspruch 1, wobei die Fixierungsvorrichtung (704) magnetisch, elektromagnetisch und/oder mechanisch (z.B. unter Verwendung einer Hebelvorrichtung) ist.

3. Maschine nach Anspruch 1 oder 2, wobei die Fixierungsvorrichtung (704) zumindest eine Schiebeklemme, einen Einschubniet, einen Gewindeniet, eine Klemme oder eine Befestigungslasche aufweist, die dazu eingerichtet sind, an einem Interface am robotischen chirurgischen System befestigt zu werden.

4. Maschine nach Anspruch 1 oder 2, wobei die Fixierungsvorrichtung (704) einen oder mehrere Ausnehmungen aufweist, die für einen oder mehrere Bolzen zum Hindurchverlaufen dimensioniert sind, um die Biegevorrichtung (500, 700) am robotischen chirurgischen System über einen oder mehrere Bolzen zu fixieren.

5. Maschine nach einem der Ansprüche 1 bis 4, wobei die Biegevorrichtung (500, 700) eine Stempelbiegevorrichtung (1210), eine 3-Rollen-Biegevorrichtung (1232), eine Kompressions-Biegevorrichtung (1242) oder eine Drehzug-Biegevorrichtung (1220) ist.

6. Maschine nach einem der Ansprüche 1 bis 4, wobei die Biegevorrichtung (500, 700) eine Stempelbiegevorrichtung (1210) ist, wobei der Kraftstempel einen ersten Gegenstempel und einen zweiten Gegenstempel aufweist, und das Biegewerkzeug ein Radiusblock ist.

7. Maschine nach einem der Ansprüche 1 bis 4, wobei die Biegevorrichtung eine 3-Rollen-Biegevorrichtung (1232) ist, wobei der Kraftstempel eine erste Gegenrolle und eine zweite Gegenrolle aufweist, und das Biegewerkzeug eine Biegerolle ist.

8. Maschine nach einem der Ansprüche 1 bis 4, wobei die Biegevorrichtung eine Kompressions-Biegevorrichtung (1242) ist, wobei der Kraftstempel ein stationäres Biegewerkzeug aufweist, wobei die Biegevorrichtung eine Klemme aufweist und das Biegewerkzeug ein Kompressionswerkzeug ist.

9. Maschine nach einem der Ansprüche 1 bis 4, wobei die Biegevorrichtung eine Drehzug-Biegevorrichtung (1220) ist, wobei der Kraftstempel einen stationären Druckstempel aufweist, die Biegevorrichtung eine Klemme, einen Folgeschlitten und ein Abstreiferwerkzeug aufweist und wobei das Biegewerkzeug ein drehbares Biegewerkzeug ist.

10. Maschine nach Anspruch 1, wobei die Biegevorrichtung eine passive Biegevorrichtung (500) ist, wobei die passive Biegevorrichtung (500) ohne die Benutzung eines Aktuators arbeitet.

11. Maschine nach Anspruch 1, wobei die Biegevorrichtung (700) einen Aktuator zum Aufbringen einer Kraft auf den Kraftstempel aufweist, was eine zwischen dem Kraftstempel und dem Biegewerkzeug positionierte Stange dazu veranlasst, sich um das Biegewerkzeug zu biegen.

12. Maschine nach Anspruch 11, wobei die Kraftübertragungsvorrichtung (504) dazu angeordnet ist, eine Kraft von einem Aktuator (506) auf den Kraftstempel (504) zu übertragen.

13. Maschine nach Anspruch 1, wobei die Biegevorrichtung (700) eine aktive Biegevorrichtung (700) ist, wobei die aktive Biegevorrichtung (700) einen oder mehrere Aktuatoren aufweist.

14. Maschine nach einem der vorangehenden Ansprüche, wobei die Biegevorrichtung eine Schneideinrichtung zum Schneiden von Stangen aufweist.

15. Maschine nach Anspruch 1, wobei die Biegevorrichtung (500, 700) zumindest teilweise ein fixiertes Modul (1002) ist.

## Revendications

1. Machine pour le cintrage peropératoire de tiges chirurgicales, la machine comprenant :
un appareil de cintrage (500, 700) pour cintrer une tige chirurgicale (1202), l'appareil de cintrage (500, 700) comprenant :
une matrice de force (1254) ;
une matrice de cintrage (1250) ; et
un dispositif de transfert de force (504) qui transfère de l'énergie d'un actionneur (506) à la matrice de force (504) provoquant ainsi le cintrage d'une tige chirurgicale (1202) positionnée entre la matrice de force (504) et la matrice de cintrage (1250) autour de la matrice de cintrage (1250) ; et **caractérisé en ce qu'**elle comprend en outre
un appareil de fixation (704) configuré pour accrocher de manière amovible l'appareil de cintrage (500, 700) à une partie d'un système chirurgical robotique qui inclut un bras de robot et une base de robot,
dans laquelle l'appareil de cintrage (500, 700) est agencé pour être relié de manière amovible au bras de robot.

2. Machine selon la revendication 1, dans laquelle l'appareil de fixation (704) est magnétique, électromagnétique, et/ou mécanique (par exemple en utilisant un dispositif de levier).

3. Machine selon la revendication 1 ou 2, dans laquelle l'appareil de fixation (704) comprend au moins l'un de clips à pousser, de rivets à pousser, de rivets à visser, de clips, et de pattes configurés pour s'attacher à une interface sur le système chirurgical robotique.

4. Machine selon la revendication 1 ou 2, dans laquelle l'appareil de fixation (704) comprend un ou plusieurs trous dimensionnés pour qu'un ou plusieurs boulons passent à travers afin d'accrocher l'appareil de cintrage (500, 700) au système chirurgical robotique via les un ou plusieurs boulons.

5. Machine selon l'une quelconque des revendications 1 à 4, dans laquelle l'appareil de cintrage (500, 700) est un appareil de cintrage à vérin (1210), un appareil de cintrage à trois rouleaux (1232), un appareil de cintrage par compression (1242), ou un appareil de cintrage par enroulement-tension (1220).

6. Machine selon l'une quelconque des revendications 1 à 4, dans laquelle l'appareil de cintrage (500, 700) est un appareil de cintrage à vérin (1210), la matrice de force comprend une première contre-matrice et une seconde contre-matrice, et la matrice de cintrage est un bloc de rayon.

7. Machine selon l'une quelconque des revendications 1 à 4, dans laquelle l'appareil de cintrage est un appareil de cintrage à trois rouleaux (1232), la matrice de force comprend un premier contre-rouleau et un second contre-rouleau, et la matrice de cintrage est un rouleau de cintrage.

8. Machine selon l'une quelconque des revendications 1 à 4, dans laquelle l'appareil de cintrage est un appareil de cintrage par compression (1242), la matrice de force comprend une matrice de cintrage stationnaire, l'appareil de cintrage comprend une pince, et la matrice de cintrage est une matrice de compression.

9. Machine selon l'une quelconque des revendications 1 à 4, dans laquelle l'appareil de cintrage est un appareil de cintrage par enroulement-tension (1220), la matrice de force comprend une matrice de pression stationnaire, l'appareil de cintrage comprend une pince, un coulisseau suiveur, et une matrice de rabattage, et la matrice de cintrage est une matrice de cintrage rotative.

10. Machine selon la revendication 1, dans laquelle l'appareil de cintrage est un appareil de cintrage passif (500), dans laquelle l'appareil de cintrage passif (500) fonctionne sans utilisation d'un actionneur.

11. Machine selon la revendication 1, dans laquelle l'appareil de cintrage (700) comprend un actionneur pour appliquer une force à la matrice de force provoquant ainsi le cintrage d'une tige positionnée entre la matrice de force et la matrice de cintrage autour de la matrice de cintrage.

12. Machine selon la revendication 11, dans laquelle le dispositif de transfert de force (504) est agencé pour transférer une force d'un actionneur (506) à la matrice de force (504).

13. Machine selon la revendication 1, dans laquelle l'appareil de cintrage (700) est un appareil de cintrage actif (700), dans laquelle l'appareil de cintrage actif (700) comprend un ou plusieurs actionneurs.

14. Machine selon l'une quelconque des revendications précédentes, dans laquelle l'appareil de cintrage comprend un couteau pour couper des tiges.

15. Machine selon la revendication 1, dans laquelle l'appareil de cintrage (500, 700) est au moins en partie un module fixe (1002).
